# EUROPEAN PATENT APPLICATION

(11) **EP 1 174 424 A1**
(43) Date of publication of application: **23.01.2002**
(21) Application number: 00917412.9
(22) Date of filing: 21.04.2000
(51) Int. Cl.: C07C 401/00, A61K 31/59, A61P 35/00, A61P 37/02, C07C 33/048, C07F 7/18

(54) **3-METHYLATED VITAMIN D DERIVATIVES**

(30) Priority: 23.04.1999 JP 11657999
(71) Applicant: CHUGAI SEIYAKU KABUSHIKI KAISHA, Tokyo, 115-8543 (JP)
(72) Inventor: TAKAYAMA, Hiroaki, Shibuya-ku, Tokyo 151-0072 (JP); FUJISHIMA, Toshie, Hachioji-shi, Tokyo 193-0834 (JP)
(74) Representative: VOSSIUS & PARTNER
(86) International application number: JP0002620
(87) International publication number: WO0064870

(57) **Abstract**

An object of the present invention is to synthesize a novel vitamin D derivative having a methyl group at the 3-position.

The present invention provides vitamin D derivatives represented by the general formula (1): 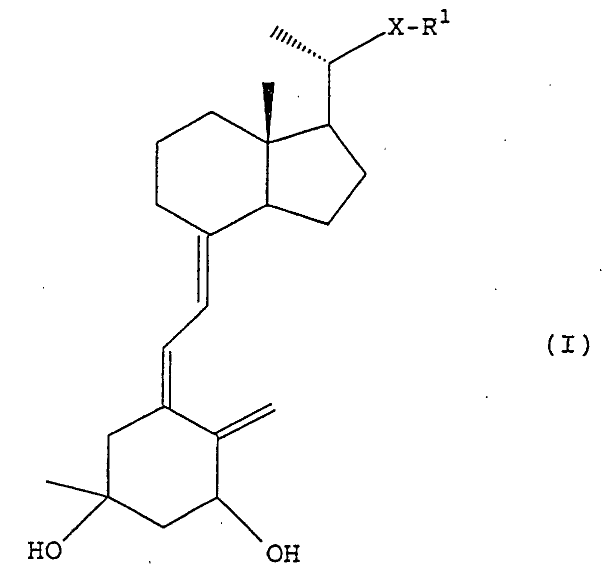 wherein X represents -CH2-, -O- or -S-; and R¹ represents a saturated or unsaturated aliphatic C₁-C₁₅ hydrocarbon group which may be substituted by 1 to 3 hydroxyl groups or protected hydroxyl groups.

## Description

### TECHNICAL FIELD

The present invention relates to a novel vitamin D derivative. More particularly, the invention relates to a vitamin D derivative having a methyl group at the 3-position.

### BACKGROUND ART

An activated vitamin D₃ including 1α,25-dihydroxy-vitamin D₃ is known to have many physiological activities such as calcium metabolism regulatory activities, growth inhibitory and differentiation inducing activities for tumor cells, and immunoregulatory activities. However, some activated vitamin D₃ disadvantageously may cause hypercalcemia during long-term and continuous administration, so that they are not suitable for use as antitumor agents, antirheumatic agents, or the like. Thus, a number of studies have been conducted to synthesize vitamin D derivatives for the purpose of separating activities of these vitamin Ds.

Introduction of a substituent in an A ring part of an activated vitamin D₃, for example, restricts possible conformation, which may produce characteristic activities. For example, 1α,25-dihydroxyvitamin D₃ having a methyl group at the 2- or 4-position is described in Bioorg. Med. Chem. Lett., 1998, 8, 151 (K.Konno et al.), ibid., 1998, 8, 2145 (T.Fujishima et al.), and Abstracts of the 118th Annual Meeting of the Pharmaceutical Society of Japan 2 p.171. However, no vitamin D₃ derivative having a methyl group at the 3-position has been reported.

### DISCLOSURE OF THE INVENTION

An object of the present invention is to provide and to synthesize a novel vitamin D₃ derivative having a methyl group at the 3-position. Another object of the present invention is to evaluate bioavailability of the vitamin D₃ derivatives having a methyl group at the 3-position.

As a result of careful studies as to achieve the above mentioned objects; the inventors of the present invention have succeeded in synthesizing a desired vitamin D derivative by employing 3-methyl-3-buten-1-ol as a starting material and by coupling a precursor of an A ring part which is obtained by stereospecificaly introducing a hydroxyl group at the 3-position by means of the Sharpless asymmetric dihydroxylation with a CD ring part which is synthesized separately using palladium catalyst; thereby achieved the present invention.

According to one aspect of the present invention, a vitamin D derivative represented by the general formula (I) is provided: 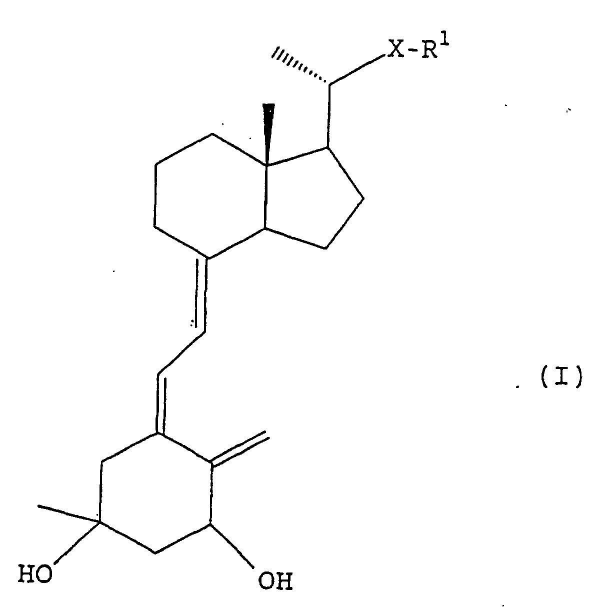 wherein X represents -CH₂-, -O- or -S-; and R¹ represents a saturated or unsaturated aliphatic C₁-C₁₅hydrocarbon group which may be substituted by 1 to 3 hydroxyl groups or protected hydroxyl groups.

Preferably, X in the general formula (I) is -CH₂-.

Preferably, R¹ in the general formula (I) is a saturated aliphatic C₁-C₁₅hydrocarbon group which is substituted by one hydroxyl group, and more preferably, by a 3-hydroxy-3-methylbutyl group.

In the present invention, 3-methyl-1β,3β-dihydroxy vitamin D₃, 3-methyl-1α,3β-dihydroxy vitamin D₃, 3-methyl-1α,3α-dihydroxy vitamin D₃, and 3-methyl-1β,3α-dihydroxy vitamin D₃ are particularly preferred.

Furthermore, according to another aspect of the present invention, a compound represented by the general formula (II) is provided: 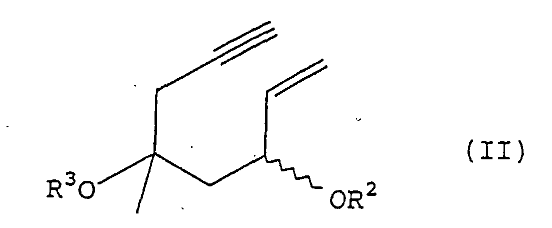 wherein R² and R³ are independently a hydrogen atom, or a protecting group for a hydroxyl group. The compound represented by the general formula (II) is a useful intermediate for synthesizing the compound represented by the general formula (I).

Furthermore, according to still another aspect of the present invention, a pharmaceutical composition comprising the vitamin D derivative represented by the general formula (I) as an active ingredient is provided, the pharmaceutical composition being a therapeutic agent for diseases accompanied with abnormal calcium metabolism, an antitumor agent, an immunomodulator, or the like.

### BEST MODE FOR CARRYING OUT THE INVENTION

Detailed modes and methods with respect to a vitamin D derivative represented by the general formula (I) and a pharmaceutical composition including thereof in accordance with the present invention are described in further detail below.

In the general formula (I), X represents -CH₂-, -O- or -S-; and R¹ represents a saturated or unsaturated aliphatic C₁-C₁₅hydrocarbon group which may be substituted by 1 to 3 hydroxyl groups or protected hydroxyl groups.

In the present application, a saturated aliphatic hydrocarbon group generally means a straight or branched alkyl group having 1 to 15 carbon atoms, with 1 to 10 carbon atoms being preferred; specific examples include methyl, ethyl, n-propyl, i-propyl, n-butyl, s-butyl, i-butyl and t-butyl groups as well as pentyl, hexyl, heptyl, octyl, nonyl and decanyl groups, with a 3-methylbutyl group, a 3-ethylpentyl group, a 4-methylpentyl group, a 3-(n-propyl)hexyl group, a 4-ethylhexyl group, a 5-methylhexyl group, a 6-methylheptyl group, a 5-ethylheptyl group, and 4-(n-propyl)heptyl group being preferred, and with a 3-methylbutyl group, a 3-ethylpentyl group, and a 4-methylpentyl group being more preferred.

In this application, an unsaturated aliphatic hydrocarbon group generally means a straight or branched C₂-C₁₅alkenyl or C₂-C₁₅alkynyl group; specific examples include a 2-propenyl group, a 2-butenyl group, a 3-butenyl group, a 2-pentenyl group, a 3-pentenyl group, a 4-pentenyl group, a 2-hexenyl group, a 3-hexenyl group, a 4-hexenyl group, a 5-hexenyl group, a 2-heptenyl group, a 3-heptenyl group, a 4-heptenyl group, a 5-heptenyl group, a 6-heptenyl group, a 2-propynyl group, a 2-butynyl group, a 3-butynyl group, a 2-pentynyl group, a 3-pentynyl group, a 4-pentynyl group, a 2-hexynyl group, a 3-hexynyl group, a 4-hexynyl group, a 5-hexynyl group, a 2-heptynyl group, a 3-heptynyl group, a 4-heptynyl group, a 5-heptynyl group, and a 6-heptynyl group, in which any hydrogen atom may be substituted by one or more alkyl groups described above, and the double bond may take either cis- or transconfiguration. Among them, 4-methyl-2-pentynyl, 4-ethyl-2-hexynyl, 4-methyl-2-pentenyl, 4-ethyl-2-hexenyl groups, and the like are preferred.

The saturated or unsaturated aliphatic hydrocarbon groups which may be substituted by a hydroxyl group means the above mentioned saturated or unsaturated hydrocarbon group in which any hydrogen atom may be substituted by one or more hydroxyl groups, for example, by 0, 1, 2, 3 hydroxyl groups, preferably by 1 or 2 hydroxyl groups, more preferably by one hydroxyl group.

Specific examples of the saturated or unsaturated aliphatic hydrocarbon groups substituted by a hydroxyl group include saturated aliphatic hydrocarbon groups such as 2-hydroxy-2-methylpropyl, 3-hydroxy-2-methylpropyl, 2,3-dihydroxy-2-methylpropyl, 2-ethyl-2-hydroxybutyl, 2-ethyl-3-hydroxybutyl, 2-ethyl-2,3-dihydroxybutyl, 2-hydroxy-2-(n-propyl)pentyl, 3-hydroxy-2-(n-propyl)pentyl, 2,3-dihydroxy-2-(n-propyl)pentyl, 2-hydroxy-3-methylbutyl, 3-hydroxy-3-methylbutyl, 4-hydroxy-3-methylbutyl, 2,3-dihydroxy-3-methylbutyl, 2,4-dihydroxy-3-methylbutyl, 3,4-dihydroxy-3-methylbutyl, 3-ethyl-2-hydroxypentyl, 3-ethyl-3-hydroxypentyl, 3-ethyl-4-hydroxypentyl, 3-ethyl-2,3-dihydroxypentyl, 3-ethyl-2,4-dihydroxypentyl, 3-ethyl-3,4-dihydroxypentyl, 2-hydroxy-3-(n-propyl)hexyl, 3-hydoxy-3-(n-propyl)hexyl, 4-hydroxy-3-(n-propyl)hexyl, 2,3-dihydroxy-3-(n-propyl)hexyl, 2,4-dihydroxy-3-(n-propyl)hexyl, 3,4-dihydroxy-3-(n-propyl)hexyl, 3-hydroxy-4-methylpentyl, 4-hydroxy-4-methylpentyl, 5-hydroxy-4-methylpentyl, 3,4-dihydroxy-4-methylpentyl, 3,5-dihydroxy-4-methylpentyl, 4,5-dihydroxy-4-methylpentyl, 4-ethyl-3-hydroxyhexyl, 4-ethyl-4-hydroxyhexyl, 4-ethyl-5-hydroxyhexyl, 4-ethyl-3,4-dihydroxyhexyl, 4-ethyl-3,5-dihydroxyhexyl, 4-ethyl-4,5-dihydroxyhexyl, 3-hydroxy-4-(n-propyl)heptyl, 4-hydroxy-4-(n-propyl)heptyl, 5-hydroxy-4-(n-propyl)heptyl, 3,4-dihydroxy-4-(n-propyl)heptyl, 3,5-dihydroxy-4-(n-propyl)heptyl, 4,5-dihydroxy-4-(n-propyl)heptyl, 4-hydroxy-5-methylhexyl, 5-hydroxy-5-methylhexyl, 6-hydroxy-5-methylhexyl, 4,5-dihydroxy-5-methylhexyl, 4,6-dihydroxy-5-methylhexyl, 5,6-dihydroxy-5-methylhexyl, 5-ethyl-4-hydroxyheptyl, 5-ethyl-5-hydroxyheptyl, 5-ethyl-6-hydroxyheptyl, 5-ethyl-4,5-dihydroxyheptyl, 5-ethyl-4,6-dihydroxyheptyl, 5-ethyl-5,6-dihydroxyheptyl, 4-hydroxy-5-(n-propyl)octyl, 5-hydroxy-5-(n-propyl)octyl, 6-hydroxy-5-(n-propyl)octyl, 4,5-dihydroxy-5-(n-propyl)octyl, 4,6-dihydroxy-5-(n-propyl)octyl, 5,6-dihydroxy-5-(n-propyl)octyl, 5-hydroxy-6-methylheptyl, 6-hydroxy-6-methylheptyl, 7-hydroxy-6-methylheptyl, 5,6-dihydroxy-6-methylheptyl, 5,7-dihydroxy-6-methylheptyl, 6,7-dihydroxy-6-methylheptyl, 6-ethyl-5-hydroxyoctyl, 6-ethyl-6-hydroxyoctyl, 6-ethyl-7-hydroxyoctyl, 6-ethyl-5, 6-hydroxyoctyl, 6-ethyl-5,7-hydroxyoctyl, 6-ethyl-6,7-hydroxyoctyl, 5-hydroxy-6-(n-propyl)nonyl, 6-hydroxy-6-(n-propyl)nonyl, 7-hydroxy-6-(n-propyl)nonyl, 5,6-dihydroxy-6-(n-propyl)nonyl, 5,7-dihydroxy-6-(n-propyl)nonyl, and 6,7-dihydroxy-6-(n-propyl)nonyl groups; and 4-hydroxy-4-methyl-2-pentenyl, 5-hydroxy-4-methyl-2-pentenyl, 4,5-dihydroxy-4-methyl-2-pentenyl, 4-ethyl-4-hydroxy-2-hexenyl, 4-ethyl-5-hydroxy-2-hexenyl, 4-ethyl-4,5-dihydroxy-2-hexenyl, 4-hydroxy-4-(n-propyl)-2-heptenyl, 5-hydroxy-4-(n-propyl)-2-heptenyl, 4,5-dihydroxy-4-(n-propyl)-2-heptenyl, 5-hydroxy-5-methyl-3-hexenyl, 6-hydroxy-5-methyl-3-hexenyl, 5,6-dihydroxy-5-methyl-3-hexenyl, 5-ethyl-5-hydroxy-3-heptenyl, 5-ethyl-6-hydroxy-3-heptenyl, 5-ethyl-5,6-dihydroxy-3-heptenyl, 5-hydroxy-5-(n-propyl)-3-octenyl, 6-hydroxy-5-(n-propyl)-3-octenyl, 5,6-dihydroxy-5-(n-propyl)-3-octenyl, 4-hydroxy-5-methyl-2-hexenyl, 5-hydroxy-5-methyl-2-hexenyl, 6-hydroxy-5-methyl-2-hexenyl, 4,5-dihydroxy-5-methyl-2-hexenyl, 4,6-dihydroxy-5-methyl-2-hexenyl, 5,6-dihydroxy-5-methyl-2-hexenyl, 5-ethyl-4-hydroxy-2-heptenyl, 5-ethyl-5-hydroxy-2-heptenyl, 5-ethyl-6-hydroxy-2-heptenyl, 5-ethyl-4,5-dihydroxy-2-heptenyl, 5-ethyl-4,6-dihydroxy-2-heptenyl, 5-ethyl-5,6-dihydroxy-2-heptenyl, 4-hydroxy-5-(n-propyl)-2-octenyl, 5-hydroxy-5-(n-propyl)-2-octenyl, 6-hydroxy-5-(n-propyl)-2-octenyl, 4,5-dihydroxy-5-(n-propyl)-2-octenyl, 4,6-dihydroxy-5-(n-propyl)-2-octenyl, 5,6-dihydroxy-5-(n-propyl)-2-octenyl, 6-hydroxy-6-methyl-4-heptenyl, 7-hydroxy-6-methyl-4-heptenyl, 6,7-dihydroxy-6-methyl-4-heptenyl, 6-ethyl-6-hydroxy-4-octenyl, 6-ethyl-7-hydroxy-4-octenyl, 6-ethyl-6,7-dihydroxy-4-octenyl, 6-hydroxy-6-(n-propyl)-4-nonenyl, 7-hydroxy-6-(n-propyl)-4-nonenyl, 6,7-dihydroxy-6-(n-propyl)-4-nonenyl, 5-hydroxy-6-methyl-3-heptenyl, 6-hydroxy-6-methyl-3-heptenyl, 7-hydroxy-6-methyl-3-heptenyl, 5,6-dihydroxy-6-methyl-3-heptenyl, 5,7-dihydroxy-6-methyl-3-heptenyl, 6,7-dihydroxy-6-methyl-3-heptenyl, 6-ethyl-5-hydroxy-3-octenyl, 6-ethyl-6-hydroxy-3-octenyl, 6-ethyl-7-hydroxy-3-octenyl, 6-ethyl-5,6-dihydroxy-3-octenyl, 6-ethyl-5,7-dihydroxy-3-octenyl, 6-ethyl-6,7-dihydroxy-3-octenyl, 5-hydroxy-6-(n-propyl)-3-nonenyl, 6-hydroxy-6-(n-propyl)-3-nonenyl, 7-hydroxy-6-(n-propyl)-3-nonenyl, 5,6-dihydroxy-6-(n-propyl)-3-nonenyl, 5,7-dihydroxy-6-(n-propyl)-3-nonenyl, 6,7-dihydroxy-6-(n-propyl)-3-nonenyl, 5-hydroxy-6-methyl-2-heptenyl, 6-hydroxy-6-methyl-2-heptenyl, 7-hydroxy-6-methyl-2-heptenyl, 5,6-dihydroxy-6-methyl-2-heptenyl, 5,7-dihydroxy-6-methyl-2-heptenyl, 6,7-dihydroxy-6-methyl-2-heptenyl, 6-ethyl-5-hydroxy-2-octenyl, 6-ethyl-6-hydroxy-2-octenyl, 6-ethyl-7-hydroxy-2-octenyl, 6-ethyl-5,6-dihydroxy-2-octenyl, 6-ethyl-5,7-dihydroxy-2-octenyl, 6-ethyl-6,7-dihydroxy-2-octenyl, 5-hydroxy-6-(n-propyl)-2-nonenyl, 6-hydroxy-6-(n-propyl)-2-nonenyl, 7-hydroxy-6-(n-propyl)-2-nonenyl, 5,6-dihydroxy-6-(n-propyl)-2-nonenyl, 5,7-dihydroxy-6-(n-propyl)-2-nonenyl, 6,7-dihydroxy-6-(n-propyl)-2-nonenyl, 4-hydroxy-4-methyl-2-pentynyl, 5-hydroxy-4-methyl-2-pentynyl, 4,5-dihydroxy-4-methyl-2-pentynyl, 4-ethyl-4-hydroxy-2-hexynyl, 4-ethyl-5-hydroxy-2-hexynyl, 4-ethyl-4,5-dihydroxy-2-hexynyl, 4-hydroxy-4-(n-propyl)-2-heptynyl, 5-hydroxy-4-(n-propyl)-2-heptynyl, 4,5-dihydroxy-4-(n-propyl)-2-heptynyl, 5-hydroxy-5-methyl-3-hexynyl, 6-hydroxy-5-methyl-3-hexynyl, 5,6-dihydroxy-5-methyl-3-hexynyl, 5-ethyl-5-hydroxy-3-heptynyl, 5-ethyl-6-hydroxy-3-heptynyl, 5-ethyl-5,6-dihydroxy-3-heptynyl, 5-hydroxy-5-(n-propyl)-3-octynyl, 6-hydroxy-5-(n-propyl)-3-octynyl, 5,6-dihydroxy-5-(n-propyl)-3-octynyl, 4-hydroxy-5-methyl-2-hexynyl, 5-hydroxy-5-methyl-2-hexynyl, 6-hydroxy-5-methyl-2-hexynyl, 4,5-dihydroxy-5-methyl-2-hexynyl, 4,6-dihydroxy-5-methyl-2-hexynyl, 5,6-dihydroxy-5-methyl-2-hexynyl, 5-ethyl-4-hydroxy-2-heptynyl, 5-ethyl-5-hydroxy-2-heptynyl, 5-ethyl-6-hydroxy-2-heptynyl, 5-ethyl-4,5-dihydroxy-2-heptynyl, 5-ethyl-4,6-dihydroxy-2-heptynyl, 5-ethyl-5,6-dihydroxy-2-heptynyl, 4-hydroxy-5-(n-propyl)-2-octynyl, 5-hydroxy-5-(n-propyl)-2-octynyl, 6-hydroxy-5-(n-propyl)-2-octynyl, 4,5-dihydroxy-5-(n-propyl)-2-octynyl, 4,6-dihydroxy-5-(n-propyl)-2-octynyl, 5,6-dihydroxy-5-(n-propyl)-2-octynyl, 6-hydroxy-6-methyl-4-heptynyl, 7-hydroxy-6-methyl-4-heptynyl, 6,7-dihydroxy-6-methyl-4-heptynyl, 6-ethyl-6-hydroxy-4-octynyl, 6-ethyl-7-hydroxy-4-octynyl, 6-ethyl-6,7-dihydroxy-4-octynyl, 6-hydroxy-6-(n-propyl)-4-nonynyl, 7-hydroxy-6-(n-propyl)-4-nonynyl, 6,7-dihydroxy-6-(n-propyl)-4-nonynyl, 5-hydroxy-6-methyl-3-heptynyl, 6-hydroxy-6-methyl-3-heptynyl, 7-hydroxy-6-methyl-3-heptynyl, 5,6-dihydroxy-6-methyl-3-heptynyl, 5,7-dihydroxy-6-methyl-3-heptynyl, 6,7-dihydroxy-6-methyl-3-heptynyl, 6-ethyl-5-hydroxy-3-octynyl, 6-ethyl-6-hydroxy-3-octynyl, 6-ethyl-7-hydroxy-3-octynyl, 6-ethyl-5,6-dihydroxy-3-octynyl, 6-ethyl-5,7-dihydroxy-3-octynyl, 6-ethyl-6,7-dihydroxy-3-octynyl, 5-hydroxy-6-(n-propyl)-3-nonynyl, 6-hydroxy-6-(n-propyl)-3-nonynyl, 7-hydroxy-6-(n-propyl)-3-nonynyl, 5,6-dihydroxy-6-(n-propyl)-3-nonynyl, 5,7-dihydroxy-6-(n-propyl)-3-nonynyl, 6,7-dihydroxy-6-(n-propyl)-3-nonynyl, 5-hydroxy-6-methyl-2-heptynyl, 6-hydroxy-6-methyl-2-heptynyl, 7-hydroxy-6-methyl-2-heptynyl, 5,6-dihydroxy-6-methyl-2-heptynyl, 5,7-dihydroxy-6-methyl-2-heptynyl, 6,7-dihydroxy-6-methyl-2-heptynyl, 6-ethyl-5-hydroxy-2-octynyl, 6-ethyl-6-hydroxy-2-octynyl, 6-ethyl-7-hydroxy-2-octynyl, 6-ethyl-5,6-dihydroxy-2-octynyl, 6-ethyl-5,7-dihydroxy-2-octynyl, 6-ethyl-6,7-dihydroxy-2-octynyl, 5-hydroxy-6-(n-propyl)-2-nonynyl, 6-hydroxy-6-(n-propyl)-2-nonynyl, 7-hydroxy-6-(n-propyl)-2-nonynyl, 5,6-dihydroxy-6-(n-propyl)-2-nonynyl, 5,7-dihydroxy-6-(n-propyl)-2-nonynyl, and 6,7-dihydroxy-6-(n-propyl)-2-nonynyl groups. Among them, 3-hydroxy-3-methylbutyl, 4-hydroxy-3-methylbutyl, 3,4-dihydroxy-3-methylbutyl, 3-ethyl-3-hydroxypentyl, 3-ethyl-4-hydroxypentyl, 3-ethyl-3,4-dihydroxypentyl, 4-hydroxy-4-methylpentyl, 5-hydroxy-4-methylpentyl, 4,5-dihydroxy-4-methylpentyl, 4-ethyl-4-hydroxyhexyl, 4-ethyl-5-hydroxyhexyl, 4-ethyl-4,5-dihydroxyhexyl, 4-hydroxy-4-methyl-2-pentenyl, 5-hydroxy-4-methyl-2-pentenyl, 4,5-dihydroxy-4-methyl-2-pentenyl, 4-ethyl-4-hydroxy-2-hexenyl, 4-ethyl-5-hydroxy-2-hexenyl, 4-ethyl-4,5-dihydroxy-2-hexenyl, 4-hydroxy-4-methyl-2-pentynyl, 5-hydroxy-4-methyl-2-pentynyl, 4,5-dihydroxy-4-methyl-2-pentynyl, 4-ethyl-4-hydroxy-2-hexynyl, 4-ethyl-5-hydroxy-2-hexynyl, and 4-ethyl-4,5-dihydroxy-2-hexynyl groups are preferred.

Examples of the protecting group for a hydroxyl group in the general formula (I) and (II) include an acyl group, a substituted silyl group, and a substituted alkyl group, with an acyl group and a substituted silyl group being preferred.

An acyl group means a substituted carbonyl group, in which a substituent;of a carbonyl group means a hydrogen atom, an optionally substituted lower alkyl group, an optionally substituted aryl group, an optionally substituted lower alkyloxy group, an optionally substituted aryloxy group, an optionally substituted aralkyloxy group, or the like. Preferably, an acyl group is a formyl group, a lower alkylcarbonyl group, an optionally substituted phenylcarbonyl group, a lower alkyloxycarbonyl group, an optionally substituted phenylalkyloxycarbonyl group, or the like, and more preferably formyl, acetyl, propionyl, butyryl, pivaloyl, benzoyl, methoxycarbonyl, ethoxycarbonyl, t-butoxycarbonyl or benzyloxycarbonyl group, or the like.

A substituted silyl group means a silyl group substituted by a lower alkyl group which may have one or more substituents, an optionally substituted aryl group, or the like, with a tri-substituted silyl group being preferred. Preferred examples of the substituted silyl group include trimethylsilyl, triethylsilyl, triisopropylsilyl, t-butyldiphenylsilyl and t-butyldimethylsilyl groups.

A substituted alkyl group means an alkyl group substituted by one or more substituents. Preferred examples of the substituents include an optionally substituted alkyloxy group and an optionally substituted aryl group, with an optionally substituted alkyloxy group being particularly preferred. Examples of the alkyl group substituted by an optionally substituted alkyloxy group such as an alkyloxy.group include, for example, methoxymethyl, 2-methoxyethoxy-methyl and tetrahydropyran-2-yl groups. Examples of the substituent include halogen atoms, and cyano, nitro, amino, hydroxyl, alkyl, alkyloxy, acyloxy and sulfonyl groups.

The stereochemstry of compounds of the general formula (I) of the present invention in which the 1-position and the 3-position are a hydroxyl group and a methyl group can occur in α or β conformation, and the respective compounds are all included within the scope of the present invention. Furthermore, when R¹ in the general formula (I) is an unsaturated aliphatic hydrocarbon group which may be optionally substituted by a hydroxyl group and includes a double bond, the present invention includes the resulting cis- and trans-isomers. All other possible enantiomers and geometric isomers are also included within the scope of the present invention.

Specifically, among compounds represented by the general formula (I) of the present invention, the most preferable compounds are 3-methyl-1β,3β-dihydroxy vitamin D₃, 3-methyl-1α,3β-dihydroxy vitamin D₃, 3-methyl-1α,3α-dihydroxy vitamin D₃, and 3-methyl-1β,3α-dihydroxy vitamin D₃.

Although there is no limitation with respect to methods for synthesizing the compounds represented by the general formula (I) of the present invention, for example, an A ring part and a CD ring part of a vitamin D derivative are separately synthesized or obtained and then subjected to coupling to form the compounds, as described in the following Examples.

A compound represented by the general formula (II), which is useful as an A ring part for synthesizing a compound represented by the general formula (I), is also a novel compound, and constitutes an aspect of the present invention. 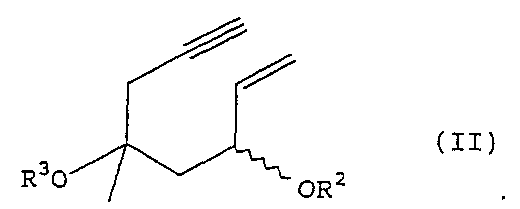 wherein R² and R³ are independently a hydrogen atom, or a protecting group for a hydroxyl group.

A method for synthesizing a compound represented by the general formula (II) will be described below.

First, a hydroxyl group of protected 3-methyl-3-buten-1-ol, a starting material, is protected by a protecting group such as a p-methoxyphenyl group, followed by the Sharpless asymmetric dihydroxylation to introduce hydroxyl groups at the 3- and 4-positions. The hydroxyl groups can be stereospecifically introduced by using either AD-mix α or AD-mix β as an reagent (the composition of AD-mix α and AD-mix β is described in J.O.C., 57, 2768 (1992), K.B.Sharpless et al.; AD-mix α contains chiral ligand (DHQ)₂PHAL (hydroquinine 1,4-phthalazinediyl diether) and AD-mix β contains chiral ligand (DHQD)₂PHAL (hydroquinidine 1,4-phthalazinediyl diether); these reagents are known as reagents for the Sharpless asymmetric dihydroxylation, and commercially available from ALDRICH and the like). After the terminal hydroxyl group is subjected to tosylation, the resulting compound is reacted with lithium acetylide to introduce a triple bond at the 4-position. The hydroxyl group at the 3-position is protected. Then, the protecting group at the 1-position is removed. After the hydroxyl group at the 1-position is oxidized to form an aldehyde, the resulting compound is reacted with vinyl magnesium bromide, followed by introducing a double bond and a hydroxyl group at the terminal. A desired A ring compound having a methyl group at the 3-position can be obtained by appropriately protecting hydroxyl groups.

On the other hand, a CD ring compound of a vitamin D derivative is known, or a desired CD ring compound can be obtained by appropriately modifying a side chain of a known CD ring compound. A CD ring compound having a -X-R¹ group as a side chain can be obtained from a known vitamin D derivative having a corresponding side chain, wherein X represents -CH₂-, -O- or -S-; and R¹ represents a saturated or an unsaturated aliphatic C₁-C₁₅hydrocarbon group which may be substituted by 1 to 3 hydroxyl groups or protected hydroxyl groups.

These known vitamin D derivatives are disclosed in Japanese Patent Publication (Kokai) Nos. 61-267550, 6-72994 and 6-256300, Japanese Patent Publication (Kohyo) Nos. 4-503669 and 4-504573, and Japanese Patent Publication (Kokai) No. 10-182597, WO 94/14766, WO 95/27697, and the like. After protecting the hydroxyl group of the above mentioned vitamin D derivative with a protecting group, the resultant compound is subjected to ozonolysis, and then, to NaBH₄ reduction, giving an alcohol having a hydroxyl group at the 8-position. An oxidization using an appropriate oxidant gives a ketone having an oxo group at the 8-position. A CD ring compound having a desired side chain can be obtained by replacing the oxo group at the 8-position with a bromomethylene group.

Coupling of an A ring compound with a CD ring compound can be carried out by known conventional methods. The CD ring compound having a bromomethylene group at the coupling site with the A ring compound obtained by the above described method is reacted with the A ring compound having a triple bond at one terminal and a double bond at the other terminal in an appropriate solvent using a palladium catalyst such as Pd₂(dba)₃ and triphenylphosphine (PPh₃). Then, the product is purified in the usual manner such as thin layer chromatography, and a protecting group for a hydroxyl group is removed, giving a desired vitamin D derivative having a methyl group at the 3-position.

The compounds of the present invention are preferably foumulated into appropriate dosage forms with pharmaceutically acceptable carriers, excipients, disintegrants, lubricants, binders, flavors, colorants, and the like. The dosage forms include tablets, granules, fine granules, capsules, powders, injections, solutions, suspensions, emulsions, percutaneous administration formulations, suppositories, and the like.

There is no restriction on routes of administration for the compounds of the present invention. The compounds may be administered orally or parenterally (intravenously, intramuscularly, intraperitoneally, percutaneously, and the like).

The dosage of the compounds of the present invention can be appropriately chosen depending on target diseases, conditions, body types, constitutions, age and sex of patients, administration routes, dosage forms, and other factors. Typically, the lower limit of dose for an adult ranges form 0.001 µg to 0.1 µg daily, and more preferable dose is about 0.01 µg. The upper limit of dose for an adult ranges from 100 µg to 10000 µg daily. More preferable dose ranges from 200 µg to 1000 µg daily, which may be divided into one to three doses.

The contents of the specification of Japanese Patent Application No. 11-116579, the application on the basis of which the present application claims priority are to be incorporated in their entirety by reference.

### EXAMPLES

The present invention will be described more specifically by way of the following Examples, which in no way limit the invention.

### Example 1: Synthesis of A ring compound for synthesizing a vitamin D derivative having a hydroxyl group at the 3β-position

A reaction scheme conducted in Example 1 is shown below.

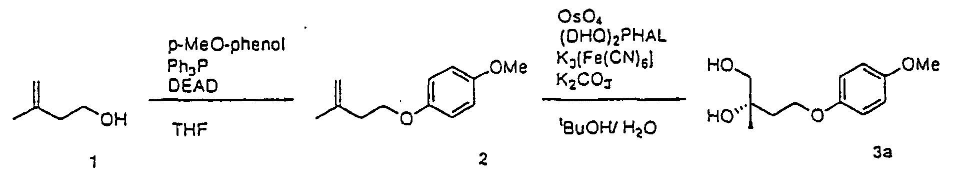

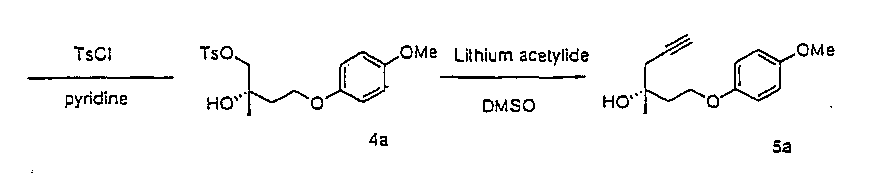

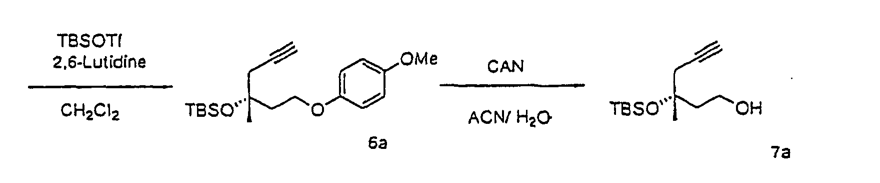

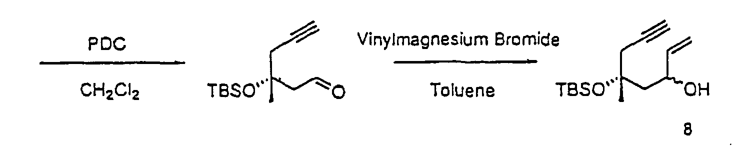

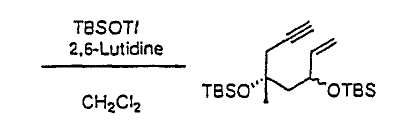

### (1) Synthesis of Compound 2

Diethylazodicarboxylic acid in toluene solution (13 mL, 30.2 mmol) at a concentration of 40% was added to a solution of 3-methyl-3-buten-1-ol (Compound 1) (2.00 g, 23.2 mmol), p-methoxyphenol (8.65 g, 69.6 mmol), and tripheriylphosphine (7.91 g, 30.2mmol) in tetrahydrofuran (50 ml), while stirring at room temperature. The reaction mixture was heated under reflux for one hour. After cooling, the solvent was distilled off under reduced pressure. The resulting residue was subjected to silica gel column chromatography (ethyl acetate:hexane=1:9) to give Compound 2 as colorless oil (4.46 g, quantitative yield).
¹H NMR(400 MHz, CDCl₃)δ 1.80(3H,s), 2.48(2H,t,J=7.0 Hz), 3.77(3H,s), 4.03(2H,t,J=6.7Hz), 4.79(1H,m), 4.84(1H,m), 6.84(4H,m);
¹³C NMR(100MHz, CDCl₃)δ 22.8(q), 37.3(t), 55.7(q), 67.2(t), 111.9(t), 114.6(d), 115.6(d), 142.3(s), 153.1(s), 153.8(s);
MS 192[M-H₂O]⁺, 124[CH₃OC₆H₄OH]⁺;
HRMS calcd.for[C₁₂H₁₆O₂] 192:1151, found 192.1156; FTIR(neat)3074, 2937, 2912, 2833, 1651, 1508, 1469, 1442, 1386, 1288, 1230, 1180, 1107, 1043, 891, 825, 738 cm⁻¹.

### (2) Synthesis of Compound 3a

A mixture of AD-mixα (ALDRICH, 14 g) solution in t-butanol (35 ml) and water (35 ml) was cooled to 0°C. To the resulting suspension, the olefin, Compound 2 (1.92 g, 10 mmol), was added. After stirring for 6 hours, sodium sulfite (15 g, 60 mmol) was added to the suspension to stop the reaction. The mixture was stirred at room temperature for 30 minutes, and extracted with ethyl acetate. The organic layer was washed with brine, dried over magnesium sulfate, and evaporated to remove the solvent. The residue was purified by the use of silica gel column chromatography (ethyl acetate:hexane=4:1) to give the desired diol, Compound 3a (1.47 g, colorless solid, 65 mol%).
¹H NMR(400MHz,CDCl₃) δ 1.25(3H,s), 1.91.(1H,ddd,J=15.0, 6.7, 4.6Hz), 2. 10(1H,ddd,J=15.0,7.9,4.6Hz), 2.50(1H,brs), 2.89(1H,brs), 3.50(2H,q,J=11.0Hz), 3.77(3H,s),
4.09(1H,ddd,J=9.8,6.7,4.6Hz), 4.11(1H,t,J=7.0Hz),
4.17(1H,ddd,J=9.8,7.9,4.6Hz), 6.84(4H,m);
¹³C NMR(100MHz, CDCl₃) δ24.1(t), 37.6(t), 55.7(q), 65.4(t), 70.0(t), 72.3(s), 114.7(d), 115.5(d), 154.3(s), 152.3(s); MS226[M]⁺, 195[M-OMe]⁺, 124[CH₃OC₆H₄OH]⁺;
HRMS calcd.for [C₁₂H₁₈O₄] 226.1205, found 226.1205; FTIR(neat) 3385, 2956, 2835, 2359, 2052, 1649, 1591, 1508, 1471, 1396, 1288, 1471, 1396, 1035, 885, 825, 733 cm⁻¹:
[α]_{D}²⁴+9.1(c = 1.05, CHCl₃);

### (3) Synthesis of Compound 4a

Under an argon atmosphere at 0°C, p-toluenesulfonyl chloride (1.71 g, 8.94 mmol) was added to a solution of Compound 3a (1.43 g, 7.45 mmol) in pyridine (15 ml). The reaction mixture was stirred for three hours at room temperature, poured into water, and extracted with ether. The organic layer was washed with 2 N hydrochloric acid and brine, dried over magnesium sulfate, and evaporated to remove the solvent. The residue was purified by the use of silica gel column chromatography (ethyl acetate:hexane=1:1) to give colorless oily Compound 4a (2.32 g, 82 mol%).
¹H NMR(400MHz, CDCl₃) δ1.25(3H,s), 1.99(2H,m), 2.44(3H,s), 2.90(1H, s), 3.77(3H,s), 3.93(2H,q,J=9.5Hz), 4.04(2H,m), 6.74-6.82(4H,m), 7.33(2H,d,J=8.2Hz), 7.79(2H,d,J=8.2Hz); ¹³C NMR(100MHz,CDCl₃), δ21.6(q), 24.5(q), 37.0(t), 55.7(q), 65.0(t), 71.1(s), 75.5(t), 114.7(d), 115.5(d), 128.0(d), 130.0(d), 132.6(s), 145.0(s), 152.2(s), 154.2(s);
MS380[M]⁺, 124[CH₃OC₆H₄OH]⁺;
HRMS calcd. for [C₁₉H₂₄O₆S] 380.1293, found 380.1294; FTIR(neat) 3526, 2953, 2835, 1597, 1508, 1466, 1358, 1292, 1230, 1176, 1097, 1074, 1037, 979, 829, 737 cm⁻¹;
[α]_{D}²⁴+8.7 (c=1.44, CHCl₃)

### (4) Synthesis of Compound 5a

Lithium acetylide-ethylenediamine complex (2.73 mg, 29.7 mmol) was added to a solution of Compound 4a (2.26 g, 5.94 mmol) in dimethyl sulfoxide (20 ml) at room temperature while stirring. The reaction mixture was stirred for 45 minutes, diluted with water, and extracted with ether. The organic layer was washed with brine, and dried over magnesium sulfate to remove the solvent. The residue was purified by the use of silica gel column chromatography (ethyl acetate:hexane=1:2) to give colorless oily Compound 5a (1.04 g, 75 mol%).
¹H NMR(400MHz, CDCl₃) δ1.37(3H,s), 2.05(1H,dt,J=14.6,
5.8Hz), 2.07(1H,t,J=2.7Hz), 2.14(1H,dt,J=14.6,5.8Hz), 2.50(1H,dd,J=16.8,1.5Hz), 2.82(1H,brs), 3.77(3H,s), 4.15(2H,t,J=5.8Hz), 6.81-6.87(4H,m);
¹³C NMR(100MHz,CDCl₃), δ26.8(q), 32.6(t), 39.1(t), 55.7(q), 65.6(t), 71.1(d), 71.6(s), 80.8(s), 114.7(d), 115.5(d), 152.5(s), 154.1(s);
MS 234[M]⁺, 216[M-H₂O]⁺, 195[M-CH₂CCH]⁺, 124[CH₃OC₆H₄OH]⁺;
HRMS calcd. for [C₁₄H₁₈O₃] 234.1256, found 234.1253; FTIR(neat)3464, 3290, 3045, 2934, 2835, 2118, 1591, 1510, 1468, 1394, 1288, 1230, 1180, 1140, 1111, 1037, 927, 887, 827, 734 cm⁻¹;
[α]_{D}²⁴-14.0 (c=0.97, CHCl₃)

### (5) Synthesis of Compound 6a

A solution of Compound 5a (1.00 g, 4.30 mmol) and 2,6-lutidine (1.5 ml, 13 mmol) in dry methylene chloride (20 ml) was stirred under an argon atmosphere at room temperature. TBSOTf (1.5 ml, 65 mmol) was added to the mixture, followed by stirring at room temperature for two hours. The mixture was diluted with methylene chloride; the organic layer was washed with water, dried over magnesium sulfate, and filtered. The filtrate was evaporated to remove the solvent; the resulting residue was subjected to silica gel column chromatography (ethyl acetate:hexane=1:10), giving colorless oily Compound 6a (1.50 g, quantitative yield).
¹H NMR(400MHz, CDCl₃) δ0.11(3H,s), 0.12(3H,s), 0.87(9H,s), 1.41(3H,s), 2.01(1H,t,J=2.7Hz), 2.02(1H,m), 2.15 (1H,quintet,J=7.0Hz), 2.40 (1H,dd,J=16.8,2.7Hz), 2.42(1H,dd,J=16.8,2.7Hz), 3.77(3H,s), 4.08(2H,t,J=7.0Hz), 6.83(4H,s);
¹³C NMR(100MHz, CDCl₃) δ -2.14(q), -2.11(q), 18.2(s), 25.7(q), 27.8(q), 33.3(t), 40.8(t), 55.7(q), 64.8(t), 70.5(s), 74.2(s), 81.5(d), 114.6(d), 115.3(d), 153.1(s), 153.7(s);
MS348[M]⁺, 309[M-CH₂CCH]⁺, 291[M-tBu]⁺;
HRMS calcd.for [C₂₀H₃₂O₃Si] 348.2121, found 348.2119; FTIR(neat)3310, 2955, 2932, 2856, 2120, 2058, 1848, 1616, 1591, 1508, 1469, 1442, 1377, 1359, 1288, 1230, 1151, 1126, 1043, 1005, 939, 833, 773, 738 cm⁻¹;
[α]_{D}²⁵ -9.3(c=1.36, CHCl₃)

### (6) Synthesis of Compound 7a

A solution of Compound 6a (1.50 g, 4.30 mmol) in acetonitrile (50 ml) and water (16 ml) were combined, stirred at 0°C, and treated with cerium diammonium nitrate (5.59 g, 10.2 mmol). Five minutes after that, ethyl acetate (80 ml) and brine (80 ml) were added to the reaction mixture to for partition. The resulting aqueous layer was extracted with ethyl acetate. The organic layers were combined, washed with a saturated sodium bicarbonate solution, dried over magnesium sulfate, and filtrated. The filtrate was evaporated to remove the solvent; the resulting residue was purified by the use of silica gel column chromatography (ethyl acetate:hexane=4:1), giving light yellow oily Compound 7a (826 mg, 79 mol%).
¹H NMR(400MHz,CDCl₃) δ0.13(3H,s), 0.14(3H,s), 0.87(9H,s), 1.40(3H,s), 1.83(1H,dt,J=14.0,6.4Hz),
1.98(1H,dt,J=14.0,6.4Hz), 2.01(1H,t,J=2.7Hz), 2.40(1H,dd,J=16.2, 2.7Hz), 2.40(1H,m),
2.51(1H,dd,J=16.2,2.7Hz), 3.83(1H,q,J=5.5Hz);
¹³C NMR(100MHz, CDCl₃) δ -2.1(q), -2.0(q), 18.0(s), 25.8(q), 27.6(q),32.7(t), 43.0(t), 59.6(t), 70.7(d), 76.2(s), 81.2(s);
MS 227[M-Me]⁺, 203[M-CH₂CCH]⁺, 185[M-^{t}Bu]⁺;
HRMS calcd.for[C₁₂H₂₃O₂Si] 227.1468, found 227.1471; FTIR(neat) 3314, 2955, 2932, 2889, 2858, 2120, 1462, 1421, 1377, 1359,1307, 1255, 1116, 1057, 1005, 939, 837, 810, 773 cm⁻¹; [α]_{D}²⁵ -8.1(c = 1.24, CHCl₃)

### (7) Synthesis of Compound 8

Compound 7a (820 mg, 3.40 mmol) and powdered molecular sieve 4 A (250 mg) were stirred in methylene chloride (10 ml) at room temperature and treated with PDC (3.19 g, 8.48 mmol). The mixture was stirred under an argon atmosphere at room temperature for seven hours. The mixture was subjected to silica gel column chromatography (ethyl acetate:hexane=1:4) to give a light yellow oily aldehyde (591 mg, 72 mol%). The obtained aldehyde was immediately subjected to the next reaction.

A solution of the above aldehyde (591 mg, 2.45 mmol) in dry toluene solution (10 ml) was stirred under an argon atmosphere at -78°C, and mixed with a solution of 1.0 M vinyl magnesium bromide in tetrahydrofuran (7 ml, 7 mmol). The reaction mixture was stirred for one hour and the reaction was stopped by the addition of a saturated ammonium chloride solution. The organic layer was extracted with ethyl acetate, washed with brine, dried over magnesium sulfate, and filtrated. The filtrate was evaporated to remove the solvent; the resulting crude mixture was subjected to silica gel column chromatography (ethyl acetate:hexane=1:5), giving alcohol Compound 8
(492 mg, 75 mol%)
¹H NMR(400 MHz,CDCl₃) δ0.16, 0.17, 0.18(6H,s), 0.88(9Hx5/9,s), 0.89(9H x4/9,s), 1.44(3Hx4/9, s), 1.46(3Hx5/9,s), 1.68(1Hx4/9,ddd,J=14.6,10.4 Hz),
1.78(1Hx5/9,ddd,J=14.6,2.1Hz),
1.90(1Hx5/9,dd,J=14.6,10.1Hz),
1.93(1Hx4/9,dd,J=14.6,2.7Hz), 2.02(1Hx4/9,t,J=2.7Hz),
2.04(1Hx5/9,t,J=2.7Hz), 2.42(1Hx4/9,dd,J=16.5,2.7Hz),
2.48(1Hx5/9,dd,J=16.5,2.7Hz), 2.53(1Hx5/9,dd,J=16.5,2.7Hz), 2.66(1Hx4/9,dd,J=16.5,2.7Hz), 3.48(1Hx5/9, m), 3.69(1Hx4/9,m), 4.44-4.52(1H,m), 5.06-5.09(1H,m), 5.29-5.30 (1H,m), 5.81-5.90 (1H,m)

### (8) Synthesis of Compound 9

A solution of Compound 8 (475 mg, 1.77 mmol) and 2,6-lutidine (0.6 ml, 8.2 mmol) in dry methylene chloride (8 ml) was stirred under an argon atmosphere at 0°C, and mixed with TBSOTf (t-butyldimethylsilyl trifurate) (0.6 ml, 2.7 mmol). The mixture was stirred at 0°C for one hour, and diluted with methylene chloride. The organic layer was washed with water, dried over magnesium sulfate, and filtered. The filtrate was evaporated to remove the solvent; the resulting residue was subjected to silica gel column chromatography (ethyl acetate:hexane=1:20), giving colorless oily Compound 9 (677 mg, quantitative yield).
¹H NMR(400MHz,CDCl₃) δ0.01, 0.03, 0.065, 0.073, 0.09, 0.11(12H,s), 0.877, 0.879, 0.884, 0.890(18H,s), 1.35(3Hx5/9,s), 1.36(3Hx4/9,s),
1.69(1Hx5/9,ddd,J=14.1,4.6Hz),
1.80(1Hx4/9,ddd,J=14.0,5.5Hz),
1.92(1Hx4/9,dd,J=14.1,6.4Hz), 1.94(1Hx5/9, dd,
J=14.3,7.0Hz), 1.97(1Hx 5/9,t,J=2.7Hz),
1.98(1Hx4/9,t,J=2.7Hz), 2.33(1Hx4/9,dd,J=16.5,2.7Hz),
2.42(1Hx5/9,dd,J=16.5,2.7Hz), 2.45(1Hx5/9,dd,J=16.5,2.7Hz), 2.51(1Hx 4/9,dd,J=16.5,2.7Hz), 4.37(1Hx4/9,q,J=6.7Hz), 4.43(1Hx5/9,m), 4.99(1Hx5/9,d,J=9.8Hz),
5.01(1Hx5/9,d,J=10.0Hz), 5.11(1Hx5/9,d,J=17.4Hz), 5.12(1Hx4/9,d,J=17.1Hz), 5.81-5.92(1H,m);
MS 367[M-Me]⁺, 343[M-CH₂CCH]⁺, 325[M-^{t}Bu]⁺;
HRMS calcd.for [C₂₀H₃₉O₂Si₂] 367.12488, found 367.2487.

### Example 2: Synthesis of A ring compound for synthesizing a vitamin D derivative having a hydroxyl group at the 3α-position

The reaction scheme conducted in Example 2 is shown below.

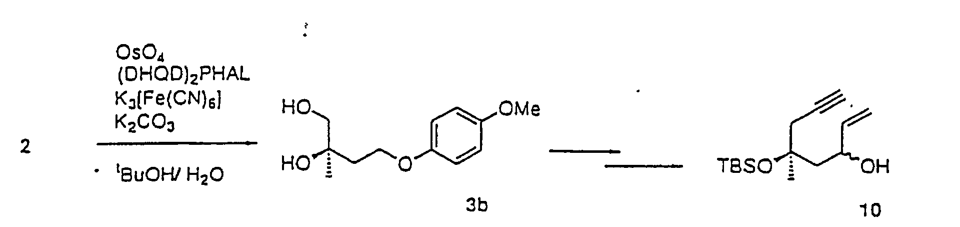

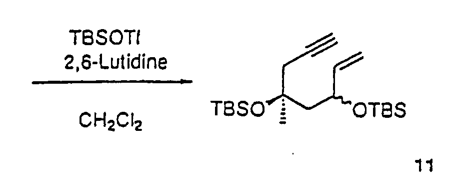

Compound 11 was synthesized from Compound 2 by the same procedure as in Example 1 except that AD-mix β was used instead of AD-mix α which was used in synthesis for Compound 3 described in Example 1 (2).

### Data on Compound 3b

[α]_{D}²⁴-8.2 (c=1.11, CHCl₃); mp 58-59°C (recry. from EA-hexane)

### Data on Compound 11

Physical property data on Compound 11 was as the same as that on Compound 9.

### Example 3: Synthesis of a vitamin D derivative having a hydroxyl group at the 3β-position

The reaction scheme conducted in Example 3 is shown thereinafter.

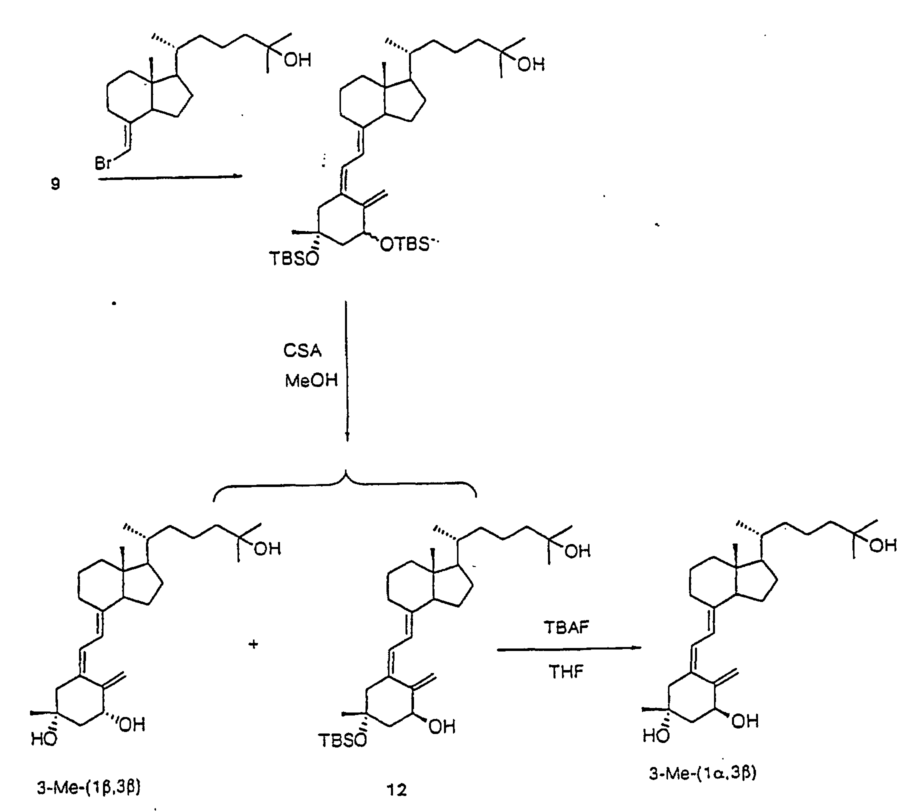

The CD ring compound (90 mg, 0.25 mmol) shown in the above reaction scheme, Compound 9 of A ring (120 mg, 1.25 equivalents) obtained in Example 1, Pd₂(dba)₃ (26 mg, 0.1 equivalent), and PPh₃ (66 mg, 1 equivalent) were refluxed in a mixture of toluene (3 ml) and triethylamine (3 ml) at 130°C for seven hours. The reaction mixture was evaporated to remove the solvent; the resulting residue was subjected to one sheet of preparative silica gel thin layer chromatography (MERCK 5744) (20×20). The chromatogram was developed using ethyl acetate/n-hexane (1:3) to give a light yellow oil (97 mg, 58 mol%).

Ninety-seven mg of the above mixture (0.15 mmol) was dissolved in methanol (4.5 ml), and mixed with 34 mg of CSA (10-Camphorsulfonic acid) (1 equivalent). The reaction was maintained for three hours at room temperature. The reaction mixture was evaporated to remove the solvent. The obtained residue was subjected to one sheet of preparative silica gel thin layer chromatography (MERCK 5744) (20×20). The chromatogram was developed using ethyl acetate/n-hexane (1:1) to give 12.5 mg of 3-methyl-1β,3β-dihydroxy vitamin D₃ (3-Me-(1β,3β) in the reaction scheme) (20%) and 24.3 mg (30 mol%) of Compound 12.

### Data on 3-Me-(1β,3β):

¹H NMR(400MHz,CDCl₃) δ0.56(3H,s), 0.94(3H,d,J=6.4Hz), 1.22(6H,s), 1.26(3H,s), 1.80(1H,dd,J=14.3,3.4Hz), 2.41(2H,s), 2.50(1H,brs), 2.84(1H,dd,J=12.2, 4.3Hz), 3.22(1H,brs), 4.42(1H,s), 5.02(1H,d,J=2.1Hz), 5.28(1H,d,J=2.1Hz), 6.07(1H,d,J=11.3Hz), 6.45(1H,d,J=11.3Hz);
¹³C NMR(100MHz, CDCl₃) δ 11.9, 14.1, 18.8, 20.8, 22.4, 23.7, 27.6, 29.1, 29.2, 29.4, 29.9, 36.1, 36.4, 40.5, 43.9, 44.4, 45.9, 50.9, 56.4, 56.5, 71.1, 71.9, 74.6, 114.5, 117.0, 125.8, 132.3, 132.3, 143.2, 146.7;
MS 430[M]⁺, 412[M-H₂O]⁺, 394[M-2H₂O]⁺, 379[M-2H₂O-Me]⁺;
HRMS calcd.for [C₂₈H₄₆O₃] 430.3447, found 430.3446; FTIR(neat)3358, 2941, 2872, 1649, 1437, 1375, 1213, 1138, 1035, 1006, 910, 817, 733 cm⁻¹:
[α]_{D}²⁴+744(c = 0.00188, EtOH); UV(EtOH) λmax 264 nm,λmin 225 nm.

### Data on Compound 12:

¹H NMR(400MHz,CDCl₃) δ0.07(3H,s), 0.08(3H,s), 0.55(3H,s), 0.78(9H,s),0.94(3H,d,J=6.4Hz), 1.21(6H,s), 1.30(3H,s), 2.27(2H,brs), 2.79(1H,m), 4.40(1H,m), 4.98(1H,t, J=2.1Hz), 5.32(1H,t,J=2.1Hz),
6.01(1H,t,J=11.3Hz), 6.17(1H,d,J=11.3Hz);
MS 544[M]⁺, 526[M-H₂O]⁺, 487[M-tBu]⁺;
HRMS calcd.for [C₃₄H₆₀O₃Si] 544.4311, found 544.4313;

Compound 12 at an amount of 24.3 mg (0.045 mmol) was dissolved in THF (5 ml), and mixed with 1 N tetrabutylammonium fluoride solution in THF (0.14 ml). The obtained mixture was heated in an oil bath at 55°C overnight. The reaction mixture was evaporated to remove the solvent; the resulting residue was subjected to one sheet of preparative silica gel thin layer chromatography (MERCK 5744) (20×20). The chromatogram was developed using ethyl acetate/n-hexane (4:1) to give 15.3mg of 3-methyl-1α,3β-dihydroxy vitamin D₃ (3-Me-(1α,3β) in the reaction scheme) (80%) as a colorless crystal.

### Data on 3-Me-(1α,3β):

¹H NMR(400MHz,CDCl₃) δ0.49(3H,s), 0.87(3H,d,J=6.7Hz), 1.15(6H,s), 1.26(3H,s), 1.97(2H,s), 2.17(1H,d,J=14.0Hz), 2.74(1H,dd,J=12.5, 4.6Hz), 4.27(1H,m), 4.95(1H,t,J=2.1Hz), 5.32(1H,t,J=2.1Hz), 5.98(1H,d,J=11.3Hz), 6.25(1H,dd,J=11.3, 1.5Hz);
¹³C NMR(100MHz, CDCl₃) δ 11.9, 14.1, 18.8, 20.8, 22.3, 22.3, 23.7, 27.6, 29.0, 29.1, 29.2, 29.4, 29.8, 36.1, 36.4, 40.5, 44.4, 46.0, 48.7, 50.3, 53.8, 56.4, 56.6, 69.5, 71.07, 71.09, 109.2, 117.0, 124.6, 134.0, 143.2, 148.4;
MS 430[M]⁺, 412[M-H₂O]⁺, 394[M-2H₂O]⁺, 379[M-2H₂O-Me]⁺;
HRMS calcd.for [C₂₈H₄₆O₃] 430.3447, found 430.3446;
FTIR(neat)3364, 2949, 2870, 2361, 1651, 1375, 1248, 1066,800 cm⁻¹:
[α]_{D}²⁵+258(c = 0.00155, EtOH); UV(EtOH)λmax 261 nm, λmin 227 nm.

### Example 4: Synthesis of a vitamin D derivative having a hydroxyl group at the 3α-position

The reaction scheme conducted in Example 4 is shown below.

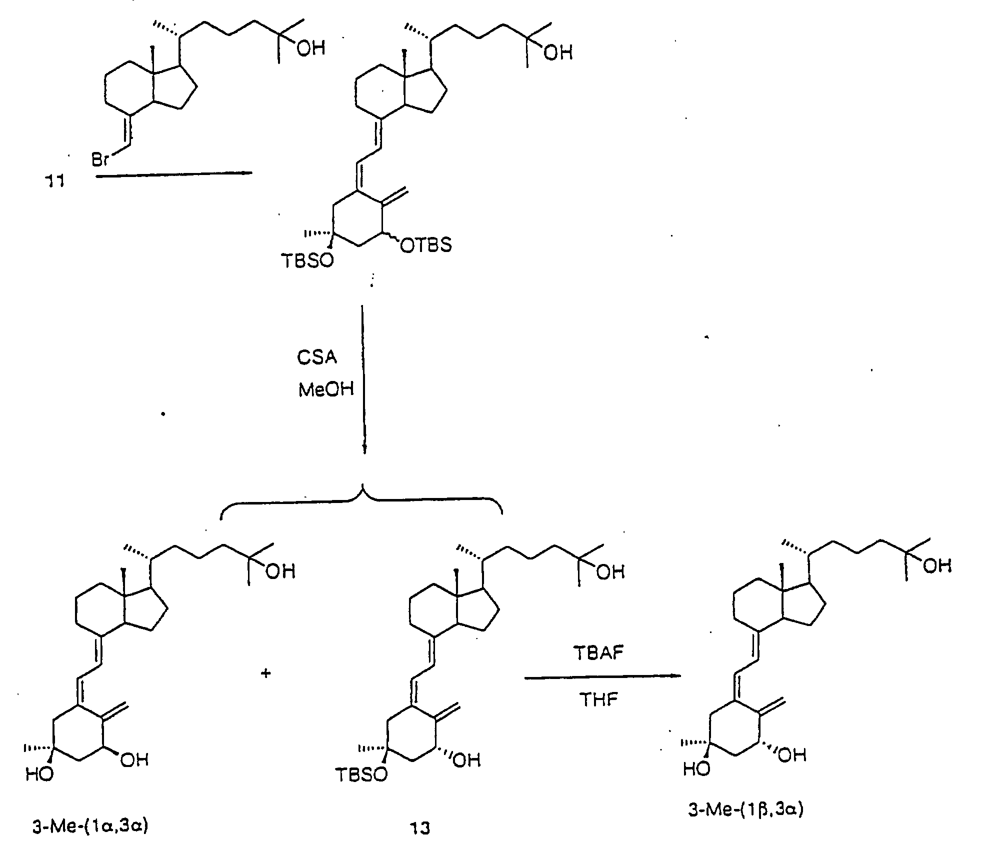

The same procedure as in Example 3 was repeated to yield 3-methyl-1α,3α-dihydroxy vitamin D₃ and 3-methyl-1β,3α-dihydroxy vitamin D₃, except that Compound 11 obtained in Example 2 was used as the A ring compound.
Data on 3-Me-(1α,3α):
¹H NMR(400MHz,CDCl₃) δ0.54(3H,s), 0.93(3H,d,J=6.4Hz), 1.21(6H,s), 1.26(3H,s), 1.82(1H,dd,J=14.3,3.4Hz), 1.99(1H,t,J=9.5Hz), 2.09(1H,dd,J=14.3, 3.7Hz), 2.40(2H,s), 2.84(1H,dd,J=12.2, 3.7Hz), 3.19(1H,brs), 4.39(1H,t,J=3.4Hz), 4.99(1H,d,J=2.1Hz), 5.26(1H,d,J=2.1Hz), 6.02(1H,d,J=11.3Hz), 6.45(1H,d,J=11.3Hz);
¹³C NMR(100MHz, CDCl₃) δ 12.1, 18.8, 20.8, 22.2, 23.4, 25.6, 27.7, 29.1, 29.2, 29.4, 29.7, 36.1, 40.6, 44.3, 44.4, 45.9, 50.9, 56.3, 56.5, 67.9, 71.1, 71.8, 74.4, 114.2, 117.1, 125.7, 132.1, 143.2, 146.7;
MS 430[M]⁺, 412[M-H₂O]⁺, 394[M-2H₂O]⁺, 379[M-2H₂O-Me]⁺;
HRMS calcd.for [C₂₈H₄₆O₃] 430.3447, found 430.3446; FTIR(neat)3362, 2943, 2872, 2363, 2241, 1649, 1456, 1375, 1213, 1134, 1035, 910, 817,733 cm⁻¹:
[α]_{D}²¹+260(c = 0.00345, EtOH); UV(EtOH)λmax 264 nm,λmin 226 nm.

### Data on Compound 13:

¹H NME(400MHz,CDCl₃) δ0.089(3H,s), 0.092(3H,s), 0.49(3H,s), 0.79(9H,s), 0.93(3H,d,J=6.4Hz), 1.22(6H,s), 1.31(3H,s), 1.98(1H,d,J=11.9Hz), 2.16(1H,dd,J=12.2, 4.9Hz), 2.27(2H,m), 2.81(1H,dd,J=12.5, 3.7Hz), 4.42(1H,m), 4.94(1H,s), 5.30(1H,t, J=2.1Hz), 6.02(1H,t,J=11.3Hz), 6.19(1H,d,J=11.3Hz);
MS 544[M]⁺, 526[M-H₂O]⁺;
HRMS calcd.for [C₃₄H₆₀O₃Si] 544.4311, found 544.4316;

### Data on 3-Me-(1β,3α):

¹H NMR(400MHz,CDCl₃) δ0.53(3H,s), 0.93(3H,d,J=6.4Hz), 1.21(6H,s), 1.31(3H,s), 1.99(2H,t,J=9.8Hz), 2.19(1H,m), 2.25(1H,dd,J=13.4, 2.1Hz), 2.42(1H,d,J=13.4Hz), 2.81(1H,dd,J=13.1, 3.7Hz), 3.29(1H,brs), 4.37(1H,m), 4.98(1H,s), 5.34(1H,s), 6.00(1H,d,J=11.0Hz), 6.33(1H,d,J=11.0Hz);
¹³C NMR(100MHz, CDCl₃) δ 12.1, 14.1, 18.8, 20.8, 22.2, 23.4, 27.7, 29.0, 29.2, 29.4, 29.8, 36.1, 36.1, 36.4, 40.5, 44.4, 45.9, 48.8, 50.4, 53.8, 56.3, 56.5, 69.4, 71.0, 71.1, 109.2, 117.1, 124.5, 133.8, 143.2, 148.4;
MS 430[M]⁺, 412[M-H₂O]⁺, 394[M-2H₂O]⁺, 379[M-2H₂O-Me]⁺;
HRMS calcd.for [C₂₈H₄₆O₃] 430.3447, found 430.3447;
FTIR(neat)3335, 2957, 2870, 2361, 1699, 1541, 1458, 1375, 1080,910 cm⁻¹:
[α]_{D}²¹+518(c = 0.00188, EtOH); UV(EtOH)λmax 261 nm,λmin 226 nm.

### Test example 1: Assay for binding to vitamin D receptor (VDR)

Bovine thymus 1α,25-dihydroxy vitamin D₃ receptor was purchased from YAMASA BIOCHEMICAL (Choshi, Chiba, Japan) and dissolved in 0.05 M phosphate buffer (pH 7.4) containing 0.3M KCl and 5mM dithiothreitol just before use. Aliquots (500 µl, 0.23 mg protein) of the receptor solution and ethanol solutions (50 µl) of 1α,25-dihydroxy vitamin D₃ or derivatives (compounds of the present invention) at various concentrations were combined, and pre-incubated at 25°C for 60 minutes. To the resultant receptor mixtures, [³H]-1α,25-dihydroxy vitamin D₃ was added at 0.1 nM, followed by standing overnight at 4°C. The mixtures were treated with dextran coated charcoal for 30 minutes at 4°C to separate the bound arid free forms of [³H]-1α,25-dihydroxy vitamin D₃, and centrifuged at 3000 rpm for ten minutes. Each of the resultant supernatants (500 µl) was mixed with ACS-II (9.5 ml) (AMERSHAM, England) for radioactivity measurement.

The binding property of the compounds of the present invention was expressed in relative values, with that of 1α,25-dihydroxy vitamin D₃ taken as 100. The results are shown below.

**(Table 1)**

| Compound | Binding property |
|---|---|
| 3-Me-(1α,3β)-dihydroxy vitamin D₃ | 18 |
| 3-Me-(1α,3α)-dihydroxy vitamin D₃ | < 0.1 |
| 3-Me-(1β,3α)-dihydroxy vitamin D₃ | < 0.1 |
| 3-Me-(1β,3β)-dihydroxy vitamin D₃ | < 0.1 |

### INDUSTRIAL APPLICABILITY

A vitamin D₃ derivative represented by the general formula (1) of the present invention is a novel compound and may be useful as a pharmaceutical agent. The compound of the present invention may be useful as reagents for studying metabolism of active vitamin D₃, that is, 1α,25-dihydroxy vitamin D₃.

## Claims

1. A vitamin D derivative represented by the general formula (I): 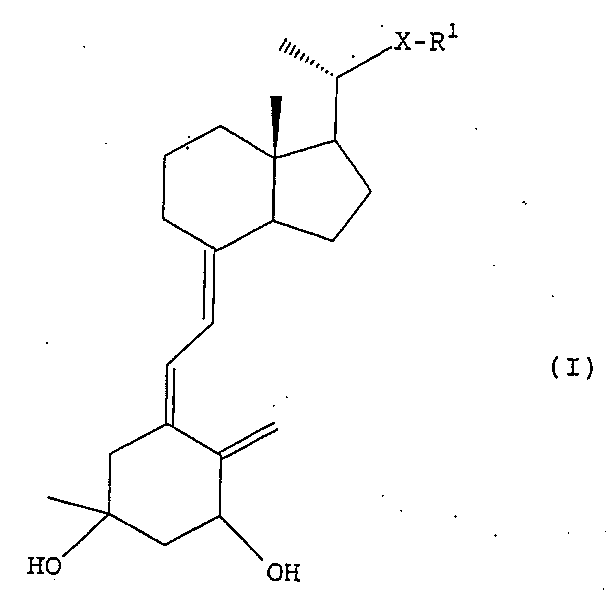 wherein
X represents -CH₂-, -O- or -S-; and
R¹ represents a saturated or unsaturated aliphatic C₁-C₁₅hydrocarbon which may be substituted by 1 to 3 hydroxyl groups or protected hydroxyl groups.

2. The vitamin D derivative according to claim 1, wherein X is -CH₂-.

3. The vitamin D derivative according to claim 1 or 2, wherein R¹ is a saturated aliphatic C₁-C₁₅ hydrocarbon group which is substituted by one hydroxyl group.

4. The vitamin D derivative according to any one of claims 1 to 3, wherein R¹ is a 3-hydroxy-3-methylbutyl group.

5. A vitamin D derivative selected from the group consisting of 3-methyl-1β,3β-dihydroxy vitamin D₃, 3-methyl-1α,3β-dihydroxy vitamin D₃, 3-methyl-1α,3α-dihydroxy vitamin D₃, and 3-methyl-1β,3α-dihydroxy vitamin D₃.

6. A compound represented by the general formula (II): 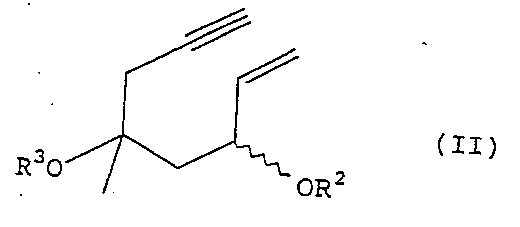 wherein R² and R³ independently represent a hydrogen atom, or a protecting group for a hydroxyl group.

7. A pharmaceutical composition comprising a vitamin D derivative according to any one of claims 1 to 5 as an active ingredient.

8. The pharmaceutical composition according to claim 7, which is a therapeutic agent for diseases accompanied with abnormality of calcium metabolism, an antitumor agent, or an immunomodulator.
